# FASCICULE DE BREVET EUROPEEN

(11) **EP 0 912 157 B1**
(45) Date de publication et mention de la délivrance du brevet: **24.11.2004**
(21) Numéro de dépôt: 97931833.4
(22) Date de dépôt: 30.06.1997
(51) Int. Cl.: A61K 7/06, A61K 38/17, G01N 33/68

(54) **HETEROMERE ISOLE LIANT L'ACIDE ARACHIDONIQUE ET SON UTILISATION EN COSMETIQUE OU PHARMACIE**
ARACHIDONSÄURE-BINDENDES ISOLIERTES HETEROMER UND SEINE KOSMETISCHE ODER PHARMAZEUTISCHE ANWENDUNG
ISOLATED ARACHIDONIC ACID-BINDING HETEROMER AND ITS USE IN COSMETICS AND PHARMACEUTICS

(30) Priorité: 02.07.1996 FR 9608219
(43) Date de publication de la demande: 06.05.1999
(73) Titulaire: L'OREAL, 75008 Paris (FR)
(72) Inventeur: SIEGENTHALER, Georges, CH-1293 Bellevue (CH)
(74) Mandataire: Tezier Herman, Béatrice
(86) Numéro de dépôt international: PCT/FR1997/001164
(87) Numéro de publication internationale: WO 1998/000095

(56) Documents cités:
- EP-A- 0 585 201
- K. ODINK: "Two calcium-binding proteins in infiltrate macrophages of rheumatoid arthritis" NATURE, vol. 330, no. 6143, 1987, pages 80-82, XP000645407 cité dans la demande
- G. SIEGENTHALER: "Purification and characterization of the human epidermal fatty acid-binding protein: localization during epidermal cell differentiation in vivo and in vitro" BIOCHEM. J., vol. 302, no. part 2, 1994, pages 363-371, XP000645424 cité dans la demande
- P. MADSEN: "Molecular cloning and expression of a novel keratinocyte protein (psoriasis-associated fatty acid-binding protein [PA-FABP]) that is highly up-regulated in psoriatic skin and that shares similarity to fatty acid-binding proteins" J. INVEST. DERMATOL., vol. 99, no. 3, 1992, pages 299-305, XP000645498
- C.D. KANE: "Expression, purification, and ligand-binding analysis of recombinant keratinocyte lipid-binding protein (MAL-1), an intracellular lipid-binding protein found overexpressed in neoplastic skin cells" BIOCHEMISTRY, vol. 35, no. 9, mars 1996, pages 2894-2900, XP000645405

## Description

L'invention concerne un hétéromère isolé liant l'acide arachidonique. Cet hétéromère peut être utilisé notamment pour stimuler et/ou induire la pousse des cheveux et/ou freiner leur chute ou pour augmenter le phénomène de la cicatrisation ou pour tester des substances modulant l'activité des acides gras ou encore comme outil de diagnostic.

Les acides gras, tels que l'acide arachidonique et ses dérivés, jouent un rôle important notamment dans la synthèse de membranes cellulaires ou nucléaires, dans le mécanisme de l'inflammation en tant que précurseurs de médiateurs et dans la fonction barrière de l'épiderme, ainsi que dans le contrôle de sa différenciation et prolifération cellulaire.

De plus, il est décrit que les acides gras modulent l'expression de nombreux gènes par le biais de récepteurs nucléaires, dénommés les PPARs (peroxisome proliferator-activated nuclear receptors).

Du fait de leur caractère labile et hydrophobe, les acides gras sont solubilisés, stabilisés et transportés dans le milieu biologique par des protéïnes de transports spécifiques cytosoliques et de bas poids moléculaires (15kDa) que l'on nomme FABP (fatty acid-binding proteins ou protéïnes se liant à des acides gras). La peau est le tissu le plus actif en ce qui concerne la synthèse de lipides. Mais, certains lipides que l'on nomme les acides gras essentiels, tels que l'acide arachidonique et l'acide linoléïque, ne peuvent être synthétisés par ces cellules et doivent donc être acquis des humeurs. Le mécanisme de capture ou de libération de ces acides gras par les cellules, et plus particulièrement par les kératinocytes, n'est pas connu. Cependant, il semble que ce mécanisme se fasse par le biais de protéïnes de transports, bien que l'identification de ces protéïnes soit jusqu'à ce jour sans succès. Récemment, dans les kératinocytes, il a été décrit une protéïne particulière (nommée la E-FABP) se liant aux acides gras épidermiques avec une forte affinité pour les acides gras présentant 18 atomes de carbone, tels que l'acide stéarique, l'acide oléïque, linoléïque et linolénique, mais avec une affinité faible ou nulle pour l'acide arachidonique et ses dérivés (Siegenthaler, G. et al. (1994) Biochem. J. 302, 363-371). Cette protéïne pourrait donc jouer un rôle important dans le transport de certains acides gras, mais pas pour celui de l'acide arachidonique et ses dérivés.

De plus, il est connu que des maladies inflammatoires, tels que l'arthrite rhumatoïque, la mucovicidose, ou des désordres dermatologiques à composante inflammatoire liée à la libération d'acide arachidonique ou de ses dérivés, tels que le psoriasis, l'eczéma, la dermatite atopique, ou encore la cicatrisation des blessures de la peau, présentent une dérégulation ou une modification du métabolisme des acides gras et plus particulièrement de l'acide arachidonique.

Dans le domaine cosmétique, on peut penser qu'une dérégulation ou une modification du métabolisme des acides gras est à l'origine des peaux sèches ou, au contraire, des peaux grasses, ou encore à une fragilisation de la peau.

Dans cette optique, on a cherché à comprendre le mécanisme de capture ou de libération et de transport des acides gras par les cellules de la peau, telles que les kératinocytes.

C'est ainsi que, de manière tout à fait surprenante, on a pu identifier et isoler, notamment à partir de kératinocytes humains, un hétéromére se liant aux acides gras, plus particulièrement à l'acide arachidonique et/ou l'acide oléïque.

Ainsi, la présente invention a pour objet un hétéromère isolé fixant l'acide arachidonique. Plus particulièrement, il fixe également l'acide oléïque.

Cet hétéromère fixe également un autre acide gras essentiel, l'acide linoléïque

Du fait de cette propriété, on voit l'intérêt que présente cet hétéromère notamment pour comprendre le mécanisme de capture, de libération ou de transport de l'acide arachidonique ou de ses dérivés et/ou pour identifier des substances susceptibles de modifier ce mécanisme de transport et donc l'activité biologique résultant de l'acide arachidonique. Par dérivés de l'acide arachidonique, on entend tous les dérivés biologiques de l'acide arachidonique, tels que notamment les hydroxyacides, les thromboxanes, les leukotriènes et les prostaglandines.

Ainsi, la présente invention a également pour objet l'utilisation de cet hétéromère.

Cet hétéromère isolé présente une constante de dissociation Kd inférieure ou égale à 300nM vis-à-vis de l'acide arachidonique, de préférence inférieure ou égale à 200nM.

Ces constantes de dissociation sont obtenues en réalisant les mesures par la technique de charbon-dextran. La technique du charbon-dextran est la suivante : des aliquots de 0,5µg d'hétéromère en solution dans 100 µl de tampon Tris (50mM Tris/HCl, 25mM NaCl, 2,5mM EDTA, 1mM DTT à pH 7,5) contenant 0,5 % de gélatine et 1 % DMSO sont incubés durant 1 heure à 37°C en présence de quantités croissantes de ligand radio-marqué, c'est-à-dire de 0-10µM pour l'acide oléique et l'acide arachidonique. Puis, chaque aliquot est traité avec 50µl de suspension de charbon-dextran T40® (5 % et 0,05 % respectivement) à 0°C durant 10 minutes, puis centrifugé à 10 000 g et la radioactivité du surnageant est mesurée. On établit une courbe de saturation et, à partir du graphique de Scatchard, on calcule le Kd.

Par ailleurs, cet hétéromère présente une constante de dissociation vis-à-vis de l'acide oléïque plus basse que celle de la E-FABP vis-à-vis du même ligand, la E-FABP étant la protéine décrite dans la publication de Siegenthaler, G. et al. (1994) Biochem. J. 302, 363-371.

L'hétéromère selon l'invention est une protéïne, celle-ci présentant un poids moléculaire d'environ 34 kDa ± 10% (analyse dans des conditions non dénaturantes (filtration sur gel)).

De manière générale, les poids moléculaires ont été déterminés par chromatographie de filtration sur colonne [(Superose 12) couplée à un HPLC]. La colonne équilibrée avec un tampon 50mM Tris/HCl contenant 0,2M de NaCl est étalonnée avec des standards protéiques de poids moléculaires allant de 6,5kDa à 150kDa, puis, un aliquot d'hétéromère est passé sur la colonne pour la détermination de son poids moléculaire. Le profil d'élution des protéïnes est analysé par la mesure de densité optique (DO) à 280nm.

Cet hétéromère peut également fixer en quantité jusqu'à 1,5 fois plus d'acide arachidonique que d'acide oléique.

En effet, lorsqu'une quantité déterminée d'hétéromère en solution est incubée en présence de 600nM d'acide arachidonique radiomarqué, puis analysée sur une colonne de filtration sur gel [(Superose 12) équilibrée avec un tampon Tris/HCl contenant 0,2M NaCl et connectée à un HPLC], le pic radioactif qui co-élue à 34 kDa et qui correspond au complexe hétéromère - acide arachidonique radio-marqué est 1,5 fois plus important que si l'expérience est réalisée dans les mêmes conditions, mais avec 600nM d'acide oléique radiomarqué.

De manière tout à fait surprenante, sous des conditions dénaturantes pour les protéines (méthode SDS-PAGE), l'analyse révèle que cet hétéromère isolé correspond à un complexe non-covalent de différentes sous-unités, constitué d'une protéine appelée MRP8 et d'une autre protéine appelée MRP14. Plus particulièrement, cet hétéromère est constitué de deux sous-unités MRP8 et une sous-unité MRP14. Ces deux protéines, MRP8 et MRP14, sont notamment décrites dans la publication de Odink et al.., (1987) Nature 330, 80-82.

Le document EP 585 201, décrit l'existence in vivo de complexes à base de MRP 8 et MRP 14 et un test de diagnostic d'états inflammatoires comprenant (a) en tant qu'antigène, les peptides recombinants MRP8, MRP14 et leurs complexes, (b) un anticorps monoclonal d'un de ses antigènes, (c) un anticorps dirigé contre l'anticorps monoclonal et un réactif lié à cet anticorps (c).
Mais ce document ne décrit ni la purification d'un hétéromère, telle que décrite dans la présente demande, ni son identification dans les kératinocytes.

Le procédé de purification de l'hétéromère correspond à des étapes classiques d'extraction protéinique à partir de tissus biologiques, plus particulièrement à partir de tissus cutanés, tels que notamment les kératinocytes de la peau.

Le procédé de purification de l'hétéromère mis en oeuvre comprend plus particulièrement les étapes suivantes : environ 5g de squames de psoriasis sont homogénéisés dans 15 ml de tampon Tris/HCl en utilisant un homogénéisateur (Polytron) puis la suspension est centrifugée à 100 000 g durant 1 heure à 4°C. Le culot est collecté et suspendu dans 5ml de même tampon et centrifugé à 10 000 g. Le surnageant est éliminé. Cette opération de lavage est effectuée 2 fois encore. Puis, le culot est traité dans 15 ml de tampon KCl (10mM Tris/HCl, 0,8M KCI, 10mM monothioglycérol, 10% glycérol, 1 mM PMSF, 10U/ml aprotinin, 10mg/ml leupeptine) à pH 7,4 durant 90 minutes à 4°C. Ce traitement est répété encore une fois. Les surnageants sont réunis et dialysés une nuit contre un tampon 20mM imidazole à pH 6, concentré en utilisant une cellule de filtration avec une membrane à dialyse de dimension de pores 3500 Da, puis la solution est chargée sur une colonne échangeuse de cations (Resource S). L'élution est effectuée par un gradient constitué du tampon de dialyse mais contenant 0,5M NaCl. La colonne est d'abord standardisée avec un échantillon incubé avec de l'acide oléique tritié. Les fractions qui co-éluent avec le pic radioactif sont collectées, dialysées contre un tampon 20mM Tris/HCl, pH 8,0 et la solution est concentrée. Cette solution est passée sur une colonne échangeuse de cations (Resource Q) équilibrée avec ce dernier tampon. L'élution sous forme de gradient s'effectue avec ce tampon Tris contenant 0,5M NaCl. De la même manière que ci-dessus, la colonne a été standardisée et seulement les protéines co-éluant avec le pic radioactif sont collectées, dialysées contre un tampon 100mM phosphate de sodium à pH 7,0 et concentré. La dernière étape de purification s'effectue sur une colonne de filtration (Superose 12) équilibrée avec le tampon phosphate. Le pic de protéine éluant à 34kDa correspond à l'hétéromère purifié.

De préférence, les kératinocytes sont des kératinocytes différenciés humains et encore plus avantageusement les kératinocytes viennent de squames psoriatiques ou de l'infundibulum (invagination épidermique) du follicule pileux.

Cet hétéromère peut être également isolé à partir de leucocytes humains. Ainsi, cet hétéromère est également présent dans les macrophages du derme de la peau se situant autour des crêtes épidermiques, ces crêtes étant caractéristiques des maladies inflammatoires de la peau, telles que le psoriasis.

La présente invention a plus particulièrement trait à une composition cosmétique ou pharmaceutique, telle que décrite dans la revendication 1.

La composition cosmétique a notamment pour but d'améliorer l'aspect cosmétique de la peau, notamment en améliorant la fonction barrière de l'épiderme.

Lorsqu'elle est pharmaceutique, la composition est de préférence dermatologique.

Elle concerne aussi l'utilisation de l'hétéromère tel que décrit ci-dessus à titre de médicament, notamment pour traiter de manière préventive ou curative la chute des cheveux ou encore pour augmenter le phénomène de la cicatrisation. En effet, sans vouloir se lier à une quelconque théorie, lors de la cicatrisation, on observe une prolifération et une différenciation cellulaire qui sont supérieures à l'état habituel (peau non lésée) et ce phénomène est concommitant à une augmentation de la quantité de l'hétéromère selon l'invention, ainsi il semble que cet hétéromère soit impliqué dans le processus de cicatrisation.

Ainsi, l'invention concerne l'utilisation de l'hétéromère tel que décrit ci-dessus dans une composition cosmétique ou à titre de médicament, ledit hétéromère ou médicament étant destiné à traiter la chute des cheveux ou à augmenter le phénomène de cicatrisation.

La quantité de l'hétéromère présent dans cette composition peut varier dans une large mesure, notamment en fonction de l'effet recherché. Pour donner un ordre de grandeur, l'hétéromère peut être présent à des concentrations comprises entre 10⁻⁶ et 20 % en poids par rapport au poids total de la composition.

La composition selon l'invention peut être administrée par voie entérale, parentérale ou topique. De préférence, on utilise la voie topique.

Par voie topique, on préfère l'application directe sur la peau, le cuir chevelu, les ongles ou les muqueuses.

Les compositions selon l'invention peuvent se présenter sous toutes les formes galéniques. Ces compositions sont préparées selon les méthodes usuelles.

Un milieu cosmétiquement ou dermatologiquement acceptable correspond généralement à un milieu compatible avec la peau, le cuir chevelu, les ongles ou les muqueuses. La composition comprenant l'hétéromère peut donc être appliquée sur le visage, le cou, les cheveux et les ongles, ou toute autre zone cutanée du corps (régions axillaires, sous-mammaires, plis du coude etc.).

Par voie topique, les compositions selon l'invention se présentent notamment sous forme de solutions aqueuses, hydroalcooliques ou huileuses, de dispersions du type lotion ou sérum, de gels anhydres ou lipophiles, d'émulsions de consistance liquide ou semi-liquide du type lait, obtenues par dispersion d'une phase grasse dans une phase aqueuse (H/E) ou inversement (E/H), ou de suspensions ou d'émulsions de consistance molle, semi-solide ou solide du type crème ou gel, ou encore de microémulsions, de microcapsules, de microparticules ou de dispersions vésiculaires de type ionique et/ou non ionique. Ces compositions sont préparées selon les méthodes usuelles.

Par voie entérale, les compositions selon l'invention peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée.

Par voie parentérale, les compositions peuvent se présenter sous forme de solutions ou de suspensions pour perfusion ou pour injection.

Elles peuvent être également utilisées pour le cuir chevelu sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.

Les quantités des différents constituants des compositions selon l'invention sont celles classiquement utilisées dans les domaines considérés.

Ces compositions constituent notamment des mousses à raser, des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires ou mieux après solaires, des lotions, gels ou mousses pour le soin de la peau, comme des lotions de nettoyage ou de désinfection, des lotions anti-solaires, des lotions de bronzage artificiel, des compositions pour le bain, des compositions désodorisantes contenant un agent bactéricide, des gels ou lotions après-rasage, des crèmes épilatoires, des compositions contre les piqûres d'insectes, des compositions anti-douleur ou des compositions pour traiter certaines maladies de la peau comme celles citées précédemment.

Les compositions selon l'invention peuvent également consister en des préparations solides constituant des savons ou des pains de nettoyage.

Les compositions peuvent aussi être conditionnées sous forme de composition pour aérosol contenant également un agent propulseur sous pression.

L'hétéromère peut être aussi incorporé dans diverses compositions pour soins ou traitements capillaires, et notamment des shampooings éventuellement antiparasitaires, des lotions de mise en plis, des lotions traitantes, des crèmes ou des gels coiffants, des compositions de teintures (notamment teintures d'oxydation) éventuellement sous forme de shampooings colorants, des lotions restructurantes pour les cheveux, des compositions de permanente (notamment des compositions pour le premier temps d'une permanente), des lotions ou des gels antichute, etc.

Les compositions de l'invention peuvent aussi être à usage bucco-dentaire, par exemple une pâte dentifrice ou un bain de bouche. Dans ce cas, les compositions peuvent contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémulsionnants utilisés dans la composition sous forme d'émulsion sont choisis parmi ceux classiquement utilisés dans les domaines cosmétique et pharmaceutique. L'émulsionnant et le coémulsionnant sont présents, dans la composition, en une proportion allant de 0,3 % à 30 % en poids, et de préférence de 0,5 à 30 % ou mieux de 0,5 à 20 % en poids par rapport au poids total de la composition. L'émulsion peut, en outre, contenir des vésicules lipidiques.

Lorsque la composition de l'invention est une solution ou un gel huileux, la phase grasse peut représenter plus de 90 % du poids total de la composition.

De façon connue, la composition de l'invention peut contenir également des adjuvants habituels dans le domaine cosmétique ou pharmaceutique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. Les quantités de ces différents adjuvants sont celles classiquement utilisées dans le domaine cosmétique ou pharmaceutique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purceilin), les huiles siliconées (cyclométhicone) et les huiles fluorées (perfluoropolyéthers). On peut aussi utiliser comme matières grasses des alcools gras, des acides gras (acide stéarique), des cires (paraffine, carnauba, cire d'abeilles).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, ou encore l'éthylcellulose, le polyéthylène.

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon et des extraits végétaux, notamment ceux d'Aloe Vera.

Comme actifs lipophiles, on peut utiliser le rétinol (vitamine A) et ses dérivés, le tocophérol (vitamine E) et ses dérivés, les céramides, les huiles essentielles.

Dans tout ce qui suit ou ce qui précède les pourcentages donnés sont exprimés en poids, sauf mention contraire, EDTA signifie l'acide éthylène diamine tétraacétique, DTT signifie le dithiothreïtol, DMSO signifie le diméthylsulfoxyde et PMSF le fluorure de phényl méthanesulfonyle.

### EXEMPLE

### Méthode de dosage de l'hétéromère

Cette méthode permet de mesurer la quantité de l'hétéromère selon l'invention présente dans les échantilleons biologiques, en excluant toute forme de contamination par les monomères le constituant, MRP8 et MRP14, ou par leurs complexes non fonctionnels (ne migrent pas). La technique consiste à séparer les protéïnes extraites d'un milieu biologique, tel que notamment les extraits tissulaires, cytosoliques, membranaires, sera, liquides synoviaux et céphalo-rachidiens, sur un gel de polyacrylamide (PAGE) en condition acide, tel qu'à un pH de 4,3. Lors de l'électrophorèse, la polarité des électrodes est inversée afin que les protéïnes chargées positivement à ce pH migrent dans ce gel. Ainsi, les protéïnes ayant un point isoélectrique très élevé, qui ne pénètrent pas dans les gels de polyacrylamide conventionnels (pH 8,8, migration vers l'anode), peuvent être analysés par cette méthode.

### Solutions pour la préparation du gel acide

A : solution pour le gel de migration pH 4,3
   24 ml de KOH 1M
   2 ml de N,N,N',N'-tétraméthyléthylènediamine (TEMED)
   8 ml d'acide acétique
   qsp à 50 ml d'eau
B : solution pour le gel de concentration pH 5,8
   24 mi de KOH 1M
   0,23 ml de TEMED
   1,44 ml d'acide acétique
   qsp à 50 ml d'eau
C : solution d'acrylamide pour le gel de migration
   6,65 g d'acrylamide
   0,1 g de N,N'-méthylène-bis-acrylamide
   qsp à 50 ml d'eau
D : solution d'acrylamide pour le gel de concentration
   2,5 g d'acrylamide
   0,625 g de N,N'-méthylène-bis-acrylamide
   qsp à 50 ml d'eau
E : solution tampon pour l'échantillon
   0,05 % rouge fuchsine, 75% glycérol
F : solution tampon pour la migration pH4,5, 4X
   31,2 g de β-alanine
   8 ml d'acide acétique
   qsp à 1 I d'eau

### Préparation du gel de migration

Entre les deux plaques de verre de l'appareil (mini-gels BioRad), on coule 3,5ml par gel du mélange constitué de : 1ml de solution A + 4,5ml de solution C + 1,5 ml H2O + 1ml d'ammonium peroxydisulfate (APS) (56mg/5ml). On laisse polymériser.

### Préparation du gel de concentration

Lorsque le gel de migration est polymérisé, on coule sur celui-ci le mélange constitué de : 1ml de solution B + 4ml de solution D + 2,25ml d'H2O + 0,75ml d'APS. On ajoute le peigne et on laisse longtemps polymériser.

Les échantillons de protéïnes sont préparées dans le tampon d'échantillon E (contenant 5µg par échantillon de cytochrome C pour améliorer la qualité de migration) et sont ensuite déposés dans les puits. Les échantillons de protéïnes peuvent être aussi incubés avec un ligand radiomarqué de l'hétéromère selon l'invention tel que l'acide oléïque tritié.

Le tampon F des réservoirs est dilué 4 fois avec de l'eau distillée et l'appareil est mis sous tension à 200V durant 45 minutes.

Cette méthode de gel acide est inspirée de Reisfald RA, Lewis UJ, Willian DE, Nature 195:281-283, 1962.

### Détection et dosage de l'hétéromère

(a) La mise en évidence de l'hétéromère peut se faire directement sur le gel en le colorant au bleu de coomassie. La bande bleue apparaissant au dessus de celle du cytochrome C correspond à celle de l'hétéromère selon l'invention.
(b) Par immunodétection sur gel, il est possible d'obtenir une grande sensibilté. Les protéïnes du gel sont alors électro-transférées sur une feuille de nitrocellulose ou de polyvinylpyrrolidone dans un tampon de 30mM phosphate de sodium à pH 6,4 sous 20V constant pendant 1 heure. Puis la membrane est incubée dans un tampon phosphate isotonique (PBS) contenant 0,5% de lait écrémé en poudre, 0,2% de Tween 20 (polysorbate). Les anticorps monoclonaux dirigés contre MRP8 et MRP14 (Biomedicals, Suisse) sont utilisés à une dilution de 1/50. Les anticorps polyclonaux dirigés contre l'hétéromère de la présente invention d'origine de lapin sont utilisés à une dilution de 1/500. Ainsi, tous ces anticorps, utilisés séparément ou ensemble, se lient à l'hétéromère selon l'invention. La bande immunoréactive de l'hétéromère selon l'invention est visualisée en utilisant des anticorps dirigés contres les anticorps cités ci-dessus venant de chèvres qui sont complexés à la peroxydase et mis en présence des substrats : 3,3'-diaminobenzidine et H2O2.

L'analyse comparative des différents puits s'effectue par densitométrie. Elle peut également s'effectuer par autoradiographie directe, lorsque l'échantillon a été incubé avec un ligand radiomarqué.

Ces trois méthodes ont été réalisées (bleu de coomassie, anticorps et acide oléïque tritié) à partir de : squasmes de psoriasis, une fraction cytosolique de squasmes de psoriasis, un extrait membranaire de squasmes de psoriasis, une fraction cytosolique de neutrophiles normaux humains et une fraction membranaire de neutrophiles normaux humains.

On constate que la bande de plus forte mobilité électrophorétique correspond à l'hétéromère selon l'invention.

Cette analyse révèle aussi que la quantité de l'hétéromère selon l'invention est plus forte dans l'extrait membranaire de squasmes de psoriasis que dans celui de fraction cytosolique de squasmes de psoriasis, l'inverse étant observé pour les neutrophiles normaux humains.

## Revendications

1. Composition cosmétique ou pharmaceutique, **caractérisée en ce qu'**elle comprend dans un milieu cosmétiquement ou pharmaceutiquement acceptable l'hétéromère correspondant à un complexe non covalent de deux sous-unités d'une protéine MRP8 et une sous-unité d'une protéine MRP-14, présentant une constante de dissociation Kd inférieure ou égale à 300nM vis-à-vis de l'acide arachidonique, ladite composition étant sous une forme choisie parmi les solutions hydroalcooliques ou huileuses, dispersions, suspensions, gels anhydres ou lipophiles, émulsions huile dans eau (H/E), émulsions E/H, microémulsions, microcapsules, microparticules ou dispersions vésiculaires de type ionique et/ou non ionique.

2. Procédé non-thérapeutique pour améliorer l'aspect cosmétique de la peau, **caractérisée en ce que** l'on applique sur la peau la composition cosmétique comprenant, dans un milieu cosmétiquement acceptable, un hétéromère correspondant à un complexe non covalent tel que défini dans la revendication précédente.

3. Hétéromère correspondant à un complexe non covalent de deux sous-unités d'une protéine MRP8 et une sous-unité d'une protéine MRP-14, présentant une constante de dissociation Kd inférieure ou égale à 300nM vis-à-vis de l'acide arachidonique, pour utilisation comme médicament.

4. Utilisation non thérapeutique de l'hétéromère correspondant à un complexe non covalent de deux sous-unités d'une protéine MRP8 et une sous-unité d'une protéine MRP-14, présentant une constante de dissociation Kd inférieure ou égale à 300nM vis-à-vis de l'acide arachidonique dans une composition cosmétique, l'hétéromère ou la composition étant destinés à traiter la chute des cheveux.

5. Utilisation de l'hétéromère correspondant à un complexe non covalent de deux sous-unités d'une protéine MRP8 et une sous-unité d'une protéine MRP-14, présentant une constante de dissociation Kd inférieure ou égale à 300nM vis-à-vis de l'acide arachidonique, pour la préparation d'un médicament destiné à traiter la chute des cheveux.

6. Utilisation de l'hétéromère correspondant à un complexe non covalent de deux sous-unités d'une protéine MRP8 et une sous-unité d'une protéine MRP-14, présentant une constante de dissociation Kd inférieure ou égale à 300nM vis-à-vis de l'acide arachidonique, pour la préparation d'un médicament destiné à augmenter le phénomène de cicatrisation.

7. Utilisation d'un hétéromère selon l'une des revendications 4 à 7 **caractérisée en ce que** l'hétéromère présente un poids moléculaire de 34 kDa ± 10 %.

8. Utilisation d'un hétéromère selon l'une des revendications 4 à 7, **caractérisé en ce que** l'hétéromère ou la composition le contenant est appliquée sur la peau, le cuir chevelu, les ongles ou les muqueuses.

## Patentansprüche

1. Kosmetische oder pharmazeutische Zusammensetzung, **dadurch gekennzeichnet, dass** sie in einem kosmetisch oder pharmazeutisch akzeptablen Medium das einem nicht kovalenten Komplex von zwei Untereinheiten eines Proteins MRP8 und einer Untereinheit eines Proteins MRP-14 entsprechende Heteromer enthält, das gegenüber Arachidonsäure eine Dissoziationskonstante Kd von 300 nM oder darunter aufweist, wobei die Zusammensetzung in einer Form vorliegt, die unter den wässrig-alkoholischen oder öligen Lösungen, Dispersionen, Suspensionen, wasserfreien oder lipophilen Gelen, Öl-in-Wasser-Emulsionen (O/W), W/O-Emulsionen, Mikroemulsionen, Mikrokapseln, Mikropartikeln oder Vesikeldispersionen vom ionischen und/oder nichtionischen Typ ausgewählt ist.

2. Nicht-therapeutisches Verfahren zur Verbesserung des Aussehens der Haut in kosmetischer Hinsicht, **dadurch gekennzeichnet, dass** auf die Haut eine kosmetische Zusammensetzung aufgetragen wird, die in einem kosmetisch akzeptablen Medium ein Heteromer enthält, das einem in dem vorhergehenden Anspruch definierten nicht kovalenten Komplex entspricht.

3. Heteromer, das einem nicht kovalenten Komplex von zwei Untereinheiten eines Proteins MRP8 und einer Untereinheit eines Proteins MRP-14 entspricht und das eine Dissoziationskonstante Kd gegenüber Arachidonsäure von 300 nM oder darunter besitzt, zur Verwendung als Arzneimittel.

4. Nicht-therapeutische Verwendung eines Heteromers, das einem nicht kovalenten Komplex von zwei Untereinheiten eines Proteins MRP8 und einer Untereinheit eines Proteins MRP-14 entspricht und gegenüber Arachidonsäure eine Dissoziationskonstante von höchstens 300 nM aufweist, in einer kosmetischen Zusammensetzung, wobei das Heteromer oder die Zusammensetzung dazu vorgesehen sind, Haarausfall zu bekämpfen.

5. Verwendung des einem nicht kovalenten Komplex von zwei Untereinheiten eines Proteins MRP8 und einer Untereinheit eines Proteins MRP-14 entsprechenden Heteromers, das gegenüber Arachidonsäure eine Dissoziationskonstante Kd von höchstens 300 nM aufweist, zur Herstellung eines Arzneimittels, das zur Behandlung von Haarausfall vorgesehen ist.

6. Verwendung des einem nicht kovalenten Komplex von zwei Untereinheiten eines Proteins MRP8 und einer Untereinheit eines Proteins MRP-14 entsprechenden Heteromers, das gegenüber Arachidonsäure eine Dissoziationskonstante Kd von höchstens 300 nM aufweist, zur Herstellung eines Arzneimittels, das dazu vorgesehen ist, die Wundheilung zu fördern.

7. Verwendung eines Heteromers nach einem der Ansprüche 4 bis 7, **dadurch gekennzeichnet, dass** das Heteromer ein Molekulargewicht von 34 kDa ± 10 % aufweist.

8. Verwendung eines Heteromers nach einem der Ansprüche 4 bis 7, **dadurch gekennzeichnet, dass** das Heteromer oder die das Heteromer enthaltende Zusammensetzung auf die Haut, die Kopfhaut, die Nägel oder die Schleimhäute aufgetragen wird.

## Claims

1. Cosmetic or pharmaceutical composition, **characterized in that** it comprises, in a cosmetically or pharmaceutically acceptable medium, the heteromer corresponding to a non-covalent complex of two subunits of an MRP8 protein and one subunit of an MRP-14 protein, with a dissociation constant Kd of less than or equal to 300 nM with respect to arachidonic acid, the said composition being in a form chosen from aqueous-alcoholic or oily solutions, dispersions, suspensions, anhydrous or lipophilic gels, oil-in-water (O/W) emulsions, W/O emulsions, microemulsions, microcapsules, microparticles or vesicular dispersions of ionic and/or nonionic type.

2. Non-therapeutic process for improving the cosmetic appearance of the skin, **characterized in that** a cosmetic composition comprising, in a cosmetically acceptable medium, a heteromer corresponding to a non-covalent complex as defined in the preceding claim is applied to the skin.

3. Heteromer corresponding to a non-covalent complex of two subunits of an MRP8 protein and one subunit of an MRP-14 protein, with a dissociation constant Kd of less than or equal to 300 nM with respect to arachidonic acid, for use as a medicament.

4. Non-therapeutic use of the heteromer corresponding to a non-covalent complex of two subunits of an MRP8 protein and one subunit of an MRP-14 protein, with a dissociation constant Kd of less than or equal to 300 nM with respect to arachidonic acid, in a cosmetic composition, the heteromer or the composition being intended to treat hair loss.

5. Use of the heteromer corresponding to a non-covalent complex of two subunits of an MRP8 protein and one subunit of an MRP-14 protein, with a dissociation constant Kd of less than or equal to 300 nM with respect to arachidonic acid, for the preparation of a medicament for treating hair loss.

6. Use of the heteromer corresponding to a non-covalent complex of two subunits of an MRP8 protein and one subunit of an MRP-14 protein, with a dissociation constant Kd of less than or equal to 300 nM with respect to arachidonic acid, for the preparation of a medicament for enhancing cicatrization.

7. Use of a heteromer according to one of Claims 4 to 7, **characterized in that** the heteromer has a molecular weight of 34 kDa ± 10%.

8. Use of a heteromer according to one of Claims 4 to 7, **characterized in that** the heteromer or the composition containing it is applied to the skin, the scalp, the nails or mucous membranes.
